# EUROPÄISCHE PATENTSCHRIFT

(11) **EP 3 184 029 B1**
(45) Veröffentlichungstag und Bekanntmachung des Hinweises auf die Patenterteilung: **29.04.2026**
(21) Anmeldenummer: 16205841.6
(22) Anmeldetag: 21.12.2016
(51) Int. Cl.: A61B 3/032, A61B 3/00

(54) **VERFAHREN ZUM ÜBERPRÜFEN DER AUGEN**
METHOD FOR TESTING EYES
PROCÉDÉ D'EXAMEN DES YEUX

(30) Priorität: 23.12.2015 DE 102015226726
(43) Veröffentlichungstag der Anmeldung: 28.06.2017
(73) Patentinhaber: Oculus Optikgeräte GmbH, 35582 Wetzlar (DE)
(72) Erfinder: STEINMÜLLER, Andreas, 35435 Wettenberg (DE); DEGLE, Stephan, 07743 Jena (DE)
(74) Vertreter: advotec.

(56) Entgegenhaltungen:
- WO-A1-2008/113853
- DE-A1- 102014 223 442
- US-A1- 2007 171 363
- US-A1- 2013 162 799
- US-A1- 2015 190 048

## Beschreibung

Die Erfindung betrifft ein Verfahren zum Überprüfen der Augen eines Probanden, mit einem Sehprüfsystem, wobei mit einem Anzeigegerät des Sehprüfsystems zumindest einem Auge des Probanden visualisierte Sehprüfzeichen angezeigt werden, wobei mit der Visualisierung der Sehprüfzeichen ein Sehtest durchgeführt wird, bei dem die Sehprüfzeichen in eine Bildwiedergabe einer realen Umweltsituation eingebettet sind, wobei das Anzeigegerät mit einem Steuergerät des Sehprüfsystems gesteuert wird, wobei das Anzeigegerät einen hintergrundbeleuchteten Bildschirm umfasst.

Derartige Sehprüfsysteme sind hinreichend bekannt und werden regelmäßig zur Durchführung von Sehtests genutzt. Zwar können Refraktionswerte eines Probanden mithilfe eines Aberrometers objektiv bestimmt werden, jedoch unterscheiden sich möglicherweise die von dem Probanden subjektiv als optimal empfundenen Refraktionswerte von den objektiv ermittelten Refraktionswerten. Unter anderem wird eine Abweichung der objektiven und subjektiven Refraktionswerte dann beobachtet, wenn eine Bestimmung der subjektiven Refraktionswerte unter mesopischen oder skotopischen Beleuchtungsbedingungen erfolgt. Insbesondere können die objektiv bei Dämmerungssehen oder Nachtsehen bestimmten Refraktionswerte von bei gleichen Beleuchtungsbedingungen subjektiv bestimmten Refraktionswerten abweichen.

Um diese Beleuchtungsbedingungen bei einem Sehtest herzustellen, werden unter anderem dem Probanden mit einem hintergrundbeleuchteten Bildschirm Sehzeichen dargeboten und gleichzeitig eine Bildschirmleuchtdichte des Bildschirms und eine Umgebungsleuchtdichte vermindert. Demnach wird der Raum, in dem der Sehtest durchgeführt wird, abgedunkelt und gleichzeitig ein Bildschirmhintergrund der Sehprüfzeichen ebenfalls dunkler dargestellt, um die gewünschten Sehbedingungen bei Dämmerungssehen oder Nachtsehen simulieren zu können. Weiter verfügen die bekannten Anzeigegeräte bzw. Bildschirme noch über ein Steuergerät, über welches eine Bedienperson eine Wiedergabe von Sehprüfzeichen auf dem Bildschirm steuern kann. Das Steuergerät kann beispielsweise in Art einer Fernbedienung ausgebildet sein. Je nach Art des Bildschirms kann dieser über eine lineare oder eine zirkulare Polarisation verfügen. Die Polarisation des Bildschirms wird regelmäßig zur Durchführung von Sehtests in Verbindung mit einer Messbrille oder einem Phoropter genutzt. Die Kameravorrichtung kann beispielsweise zur Vermessung der Augen genutzt werden, wobei bei simulierten Dämmerungssehen oder Nachtsehen eine Vermessung aufgrund der Beleuchtungsbedingungen kaum möglich ist.

Bildschirme, die ihre Bildschirmleuchtdichte bzw. Hintergrundleuchtdichte in Abhängigkeit einer Umgebungsleuchtdichte anpassen können, sind bereits bekannt. So verfügen beispielsweise einige Mobiltelefone über diese Funktion. Bei den bekannten Bildschirmen wird mittels eines optoelektronischen Sensors auf den Sensor einfallendes Licht bzw. eine Umgebungsleuchtdichte gemessen, und eine Bildschirmleuchtdichte des Bildschirms über die Hintergrundbeleuchtung des Bildschirms geregelt bzw. entsprechend angepasst. Bei beispielsweise sich erhöhender Umgebungsleuchtdichte wird auch eine Bildschirmleuchtdichte erhöht und umgekehrt. Derartige Anpassungsvorrichtungen weisen jedoch den Nachteil auf, dass die diskreten elektronischen Bauteile der Anpassungsvorrichtung, wie beispielsweise der optoelektronische Sensor, hinsichtlich ihres Ansprechverhaltens regelmäßig eine nicht lineare Kennlinie aufweisen. Die Anpassung der Bildschirmleuchtdichte an eine Umgebungsleuchtdichte erfolgt damit nicht proportional, sondern entsprechend einer Funktion, die durch die Funktionen der jeweiligen elektronischen Bauteile der Anpassungsvorrichtung bestimmt ist. Diese nicht lineare Funktion der Anpassung der Bildschirmleuchtdichte an die Umgebungsleuchtdichte verfälscht daher ein Ergebnis der subjektiven Refraktionsbestimmung bei wechselnden, unterschiedlichen Beleuchtungsbedingungen.

Aus der US 2007/171363 A1 ist ein Sehprüfsystem mit den Merkmalen des Oberbegriffs des unabhängigen Anspruchs bekannt. Das Sehprüfsystem umfasst einen LCD-Monitor zur Darstellung von Sehzeichen oder sonstigen Darstellungen, wie beispielsweise Abbildungen von Tieren. Benachbart dem LCD-Monitor ist ein Spiegel angeordnet, der mit einer Beleuchtungsvorrichtung und einer Kameravorrichtung zusammenwirkt, derart, dass über den Spiegel Augen eines Probanden, der auf den LCD-Monitor blickt, mit Infrarotlicht beleuchtet und mit einer Kamera aufgenommen werden können. Dadurch soll unter anderem eine Bewegung der Augen und ein Pupillendurchmesser bestimmt werden können. Eine Kamera beziehungsweise ein Detektor und IR-Leuchtioden, die die Kameravorrichtung und die Beleuchtungsvorrichtung ausbilden, sind unterhalb des Bildschirms fest montiert.

Die WO 2008/113853 A1 beschreibt ein Sehprüfsystem, welches einen LCD-Monitor und eine Vorrichtung zur Kalibrierung des LCD-Monitors umfasst. Die Vorrichtung umfasst zumindest zwei Sensoren, mit denen eine Beleuchtungsstärke in einer Umgebung sowie eine Bildschirmhelligkeit und Farbe detektiert werden kann. Weiter ist vorgesehen, eine Helligkeit des Bildschirms an eine Umgebungshelligkeit anzupassen. Dabei ist zunächst eine sogenannte Selbstkalibrierung des Bildschirms vorgesehen, bei der eine Helligkeit des Bildschirms mittels des einen Sensors, welcher direkt vor dem Bildschirm angeordnet ist, geregelt wird.

Ein weiteres Sehprüfsystem ist aus der DE 10 2014 223442 A1 bekannt. Es offenbart eine infrarotgestützte Augenuntersuchung mit IR-Lichtquellen und IR-Kameras zur Pupillenmessung unter verschiedenen Lichtverhältnissen.

Der vorliegenden Erfindung liegt daher die Aufgabe zugrunde, ein Verfahren zum Überprüfen der Augen eines Probanden mit einem Sehprüfsystem vorzuschlagen, durch das genauere Ergebnisse bei Sehtests schnell erzielbar sind.

Diese Aufgabe wird durch ein Verfahren mit den Merkmalen des Anspruchs 1 gelöst.

Bei dem erfindungsgemäßen Verfahren zum Überprüfen der Augen eines Probanden mit einem Sehprüfsystem werden mit einem mit einem Anzeigegerät des Sehprüfsystems zumindest einem Auge des Probanden visualisierte Sehprüfzeichen angezeigt, wobei mit der Visualisierung der Sehprüfzeichen ein Sehtest durchgeführt wird, bei dem die Sehprüfzeichen in eine Bildwiedergabe einer realen Umweltsituation eingebettet sind, wobei das Anzeigegerät mit einem Steuergerät des Sehprüfsystems gesteuert wird, wobei das Anzeigegerät einen hintergrundbeleuchteten Bildschirm umfasst, wobei das Anzeigegerät eine Beleuchtungsvorrichtung mit einer Infrarotlichtquelle aufweist, wobei das Anzeigegerät eine zur Aufnahme von Infrarotlicht angepasste Kameravorrichtung aufweist, wobei mittels der Infrarotlichtquelle Augen des Probanden mit Infrarotlicht beleuchtet und mittels der Kameravorrichtung unabhängig von einer Umgebungsbeleuchtung aufgenommen werden, wobei objektiv gemessene Refraktionswerte des Probanden an das Steuergerät übermittelt oder in dieses eingegeben werden, wobei das Steuergerät die Größe der darzustellenden Sehprüfzeichen in Abhängigkeit der objektiven Refraktionswerte automatisch auswählt, wobei die Sehprüfzeichen in einer Größe visualisiert werden, die an die objektiv gemessenen Refraktionswerte des Probanden angepasst sind, wobei eine subjektive Überprüfung der objektiv gemessenen Refraktionswerte erfolgt.

Erfindungsgemäß wird mit der Visualisierung der Sehprüfzeichen ein Sehtest durchgeführt, bei dem die Sehprüfzeichen in eine Bildwiedergabe einer realen Umweltsituation eines Probanden eingebettet sind. Die reale Umweltsituation kann beispielsweise eine Darstellung einer Landschaft sein, aus der sich eine perspektivische Ansicht der Landschaft ergibt. Innerhalb dieser Landschaft können die Sehprüfzeichen dargestellt werden.

Erfindungsgemäß werden die Sehprüfzeichen in einer Größe visualisiert, die an objektiv gemessene Refraktionswerte eines Probanden angepasst ist, wobei eine subjektive Überprüfung der objektiv gemessenen Refraktionswerte erfolgt. Ein direkter Vergleich zu objektiv und zu subjektiv gemessenen Refraktionswerten wird möglich, wenn die Sehprüfzeichen in einer Größe visualisiert werden, die an die objektiv gemessenen Refraktionswerte des Probanden angepasst ist. Dazu kann vorgesehen sein, dass die objektiv gemessenen Refraktionswerte an das Steuergerät übermittelt oder in dieses eingegeben werden, wobei das Steuergerät dann die Größe der darzustellenden Sehprüfzeichen in Abhängigkeit der objektiven Refraktionswerte automatisch auswählt.

Mittels der Kameravorrichtung sind die Augen des Probanden vollständig oder teilweise in verschiedenen Spektren aufnehmbar. Die Kameravorrichtung kann eine digitale Kamera bzw. ein Kamerachip mit einem Objektiv sein, der in einem Rahmen des Bildschirms integriert ist. Bei einer Aufnahme der Augen des Probanden können mit der Kameravorrichtung unter anderem ein beleuchtungsabhängiger Pupillendurchmesser von Augen des Probanden erfasst und gemessen werden. Diese Informationen können im Rahmen von Sehtests weiter genutzt werden.

Mittels der Beleuchtungsvorrichtung mit der Infrarotlichtquelle, können Augen des Probanden beleuchtet werden. Insbesondere dann, wenn Sehtests bei mesopischen oder skotopischen Lichtbedingungen durchgeführt werden, ist es aufgrund einer verminderten Umgebungshelligkeit schwierig, mit der Kameravorrichtung Augen eines Probanden zur Durchführung bestimmter Sehtests aufzunehmen. Mit der Infrarotlichtquelle können die Augen des Probanden unabhängig von einer Umgebungsbeleuchtung mit Infrarotlicht beleuchtet und mittels der entsprechend angepassten Kameravorrichtung aufgenommen werden. Vorteilhaft kann so auch eine Blendung des Probanden durch die Beleuchtung der Augen mit Infrarotlicht vermieden werden. Auch kann vorgesehen sein, dass die Beleuchtungsvorrichtung mehrere Infrarotlichtquellen, beispielsweise IR-Leuchtdioden, umfasst. Die Infrarotlichtquellen können unmittelbar benachbart einer Kameravorrichtung angeordnet sein. Vorzugsweise können dann zwei Infrarotlichtquellen in einer Ebene mit den Augen des Probanden neben der Kameravorrichtung relativ zu dieser äquidistant angeordnet sein. Auch ist es möglich, Infrarotlichtquellen im Rahmen des Anzeigegeräts anzuordnen. Insgesamt wird es so möglich festzustellen, ob die objektiv bei Dämmerungssehen oder Nachtsehen bestimmten Refraktionswerte von bei gleichen Beleuchtungsbedingungen und damit gleichen Pupillendurchmessern subjektiv bestimmten Refraktionswerten abweichen. Prinzipiell kann der Bildschirm auch beispielsweise mit linearer oder zirkularer Polarisation, oder einer anderen Einrichtung, die zur Bildtrennung nutzbar ist, ausgebildet sein.

Auch kann die Kameravorrichtung und/oder die Infrarotlichtquelle in eine Verwahrposition im Anzeigegerät oder in eine Aufnahmeposition außerhalb des Anzeigegeräts bewegbar ausgebildet sein. Weiter kann die Kameravorrichtung und/oder die Infrarotlichtquelle an dem Bildschirm angeordnet sein, so dass die Kamera nach Bedarf in eine Verwahrposition, beispielsweise hinter dem Bildschirm, versenkt oder für eine Kameraaufnahme in eine Aufnahmeposition neben dem Bildschirm verbracht werden kann. Eine Bewegung der Kamera von der Verwahrposition in die Aufnahmeposition und zurück kann durch eine Antriebseinheit der Kameravorrichtung erfolgen. Wenn eine vergleichsweise große Kamera verwendet wird, kann ein Umlenkprisma vorgesehen sein, so dass die Kamera platzsparend hinter dem Bildschirm angeordnet werden kann.

Der Bildschirm kann eine Anpassungsvorrichtung zur Anpassung einer Bildschirmleuchtdichte des Bildschirms an eine Umgebungsleuchtdichte aufweisen, wobei die Anpassungsvorrichtung eine Messeinrichtung zur Messung der Bildschirmleuchtdichte aufweisen kann. Insbesondere dadurch, dass die Anpassungsvorrichtung die Messeinrichtung aufweisen kann, mit der die Bildschirmleuchtdichte des Bildschirms gemessen werden kann, wird es möglich, eine Hintergrundbeleuchtung des Bildschirms und damit die Bildschirmleuchtdichte zu regeln. Mit der Messeinrichtung kann stets kontrolliert werden, ob die gemessene Bildschirmleuchtdichte einer vorausgesetzten Bildschirmleuchtdichte bei einer Umgebungsleuchtdichte entspricht. Bei einer Abweichung der gemessenen Bildschirmleuchtdichte von der vorausgesetzten Bildschirmleuchtdichte kann eine entsprechende Korrektur der Bildschirmleuchtdichte durch die Anpassungsvorrichtung erfolgen, so dass die gemessene Bildschirmleuchtdichte der vorausgesetzten Bildschirmleuchtdichte entspricht. Folglich kann unabhängig von möglichen Kennlinien diskreter elektronischer Bauteile des Anzeigegerätes mit der Anpassungsvorrichtung eine Anpassung der Bildschirmleuchtdichte an eine Umgebungsleuchtdichte proportional bzw. entsprechend einer linearen Kennlinie erfolgen, wodurch Sehtests aufgrund der gleichbleibenden Darstellungsbedingungen von Sehprüfzeichen bei unterschiedlicher Umgebungsleuchtdichte noch genauer durchgeführt werden können.

In einer Ausführungsform kann die Messeinrichtung einen optoelektronischen Sensor aufweisen, der benachbart oder vor einer Anzeigefläche des Bildschirms angeordnet sein kann, derart, dass die Bildschirmleuchtdichte messbar ist. Der optoelektronische Sensor kann beispielsweise in einer Längsseite oder Ecke eines Rahmens des Bildschirms angeordnet sein, derart, dass vom Bildschirm emittiertes Licht auf den optoelektronischen Sensor fällt. Dabei kann der optoelektronische Sensor so angeordnet sein, dass er nicht unmittelbar vor der Anzeigefläche des Bildschirms an dieser anliegt, sondern lediglich an die Anzeigefläche angrenzt. Es kann aber auch vorgesehen sein, dass der optoelektronische Sensor unmittelbar vor der Anzeigefläche, in einem Abstand von der Anzeigefläche oder unmittelbar an dieser anliegend angeordnet ist. Durch diese Anordnung des optoelektronischen Sensors wird es möglich, eine Bildschirmleuchtdichte des Bildschirms vergleichsweise genau zu messen, ohne dass der optoelektronische Sensor visuell störend vor der Anzeigefläche sichtbar ist.

Die Messeinrichtung kann einen weiteren optoelektronischen Sensor aufweisen, mit dem die Umgebungsleuchtdichte messbar ist. Der weitere optoelektronische Sensor kann ebenfalls in einem Rahmen des Bildschirms oder an einer anderen Stelle des Anzeigegeräts angeordnet sein. Wesentlich ist, dass vom Bildschirm emittiertes Licht nicht auf den weiteren optoelektronischen Sensor fällt, da dies ein Messergebnis verfälschen könnte. Insgesamt wird es dann möglich, die Umgebungsleuchtdichte und die Bildschirmleuchtdichte zu messen und entsprechend zu regeln.

Das Sehprüfsystem kann als ein Anzeigegerät ein ortfestes Ferntest-Anzeigegerät aufweisen, dessen Anzeigeflächengröße für Sehtests mit einem Sehabstand von 3 m bis 10 m, vorzugsweise von 4 m bis 8 m ausgebildet ist, und/oder als ein Anzeigegerät ein mobil handhabbares Nahtest-Anzeigegerät aufweisen, dessen Anzeigeflächengröße für Sehtests mit einem Sehabstand von 10 cm bis 3 m, vorzugsweise von 30 cm bis 1 m ausgebildet ist. Das Ferntest-Anzeigegerät kann dann zur Darstellung von Sehprüfzeichen zum Testen des Fernsehens und das Nahtest-Anzeigegerät zur Darstellung von Sehprüfzeichen zum Testen des Nahsehens genutzt werden. Das Sehprüfsystem kann entweder das Ferntest-Anzeigegerät oder das Nahtest-Anzeigegerät sowie gegebenenfalls weitere Anzeigegeräte oder auch das Ferntest-Anzeigegerät und das Nahtest-Anzeigegerät und gegebenenfalls weitere Anzeigegeräte aufweisen. Das Ferntest-Anzeigegerät kann vorzugsweise in dem oben angegebenen Sehabstand relativ zu dem Probanden ortsfest aufgestellt oder an einer Wand montiert sein. Wenn der Proband relativ zum Ferntest-Anzeigegerät in einer definierten Sehentfernung zur Durchführung von Sehtestes platziert wird, kann die Sehentfernung zu dem Ferntest-Anzeigegerät genau bestimmt werden. Auch kann dann eine Anzeigeflächengröße des Ferntest-Anzeigegeräts um ein Vielfaches größer sein als eine Anzeigeflächengröße des Nahtest-Anzeigegeräts, da gegebenenfalls auf der Anzeigefläche des Ferntest-Anzeigegeräts vergleichsweise größere Sehprüfzeichen dargestellt werden. Dadurch, dass das Nahtest-Anzeigegerät mobil handhabbar ist, kann dieses in einem nahezu beliebigen Abstand innerhalb des oben angegebenen Sehabstands relativ zu den Augen des Probanden von einer Bedienperson oder dem Proband gehalten oder platziert werden. Entsprechende Sehtests können so in unterschiedlichsten Sehabständen zu dem Nahtest-Anzeigegerät durchgeführt werden. Sowohl das Ferntest-Anzeigegerät als auch das Nahtest-Anzeigegerät können mit dem Steuergerät von einer Bedienperson ferngesteuert werden.

Das Steuergerät kann ein Mobiltelefon oder ein Tablet-Computer sein. Diese Steuergeräte verfügen über einen berührungssensitiven Bildschirm, über den eine Bedienperson bequem unterschiedliche Sehtests oder Sehprüfzeichen auswählen und dem Anzeigegerät zur Darstellung zuweisen kann. Weiter ist es möglich, das Steuergerät so zu programmieren, dass die Steuerung des Anzeigegeräts vollständig von dem Steuergerät durchgeführt wird, d. h. das Anzeigegerät dient dann lediglich zur Darstellung der vom Steuergerät initiierten Sehprüfzeichen. Unabhängig vom Anzeigegerät ist es dann auch möglich, über eine Softwareaktualisierung des Steuergeräts Änderungen der Sehprüfzeichen oder neue Sehtests in das Steuergerät einzuspielen, ohne dass dies beim Anzeigegerät erforderlich wäre. Das Steuergerät kann mittels WLAN oder Bluetooth drahtlos mit dem Anzeigegerät kommunizieren. Das Steuergerät kann jedoch auch ein fest installierter Computer oder ein Laptop sein, auf dem eine Software zur Steuerung des Anzeigegeräts ausgeführt werden kann.

Weiter kann das Anzeigegerät eine Blendvorrichtung aufweisen, mittels der Augen des Probanden beleuchtbar sind. Die Blendvorrichtung kann zumindest eine Lichtquelle umfassen, die benachbart dem Bildschirm angeordnet ist. Beispielsweise kann die Lichtquelle eine Leuchtdiode sein. Die Lichtquelle kann in einem Rahmen des Anzeigegeräts integriert sein. Weiter kann vorgesehen sein, an jeder Längsseite des Bildschirms des Anzeigegeräts eine Lichtquelle der Blendvorrichtung anzuordnen.

Das Sehprüfsystem kann einen Phoropter oder eine Messbrille umfassen. Dann wird es möglich, jeweils eine Refraktion von Augen eines Probanden zu bestimmen. Auch kann der Phoropter oder die Messbrille Farbfilter oder Polarisationsfilter aufweisen, die jeweils an eine Farbdarstellung und/oder eine Polarisation des Bildschirms angepasst sind, so dass monokulare und binokulare Sehtests durchgeführt werden können. Wenn beispielsweise bereits ein Phoropter oder eine Messbrille mit linearer oder zirkularer Polarisation vorhanden ist, kann das Anzeigegerät des Sehprüfsystems so ausgewählt werden, dass dieses überstimmend mit dem Phoropter oder der Messbrille polarisiert ist. Eine Korrektur einer Polarisation, beispielsweise mit einer λ/4-Folie, ist somit nicht erforderlich.

Darüber hinaus kann das Sehprüfsystem eine haustechnische Einrichtung zur Lichtsteuerung umfassen, die mit dem Steuergerät steuerbar ist. Die haustechnische Einrichtung kann beispielsweise eine elektrisch betätigbare Jalousie, ein Rollladen und/oder eine künstliche Beleuchtung eines Innenraums sein, in dem das Sehprüfsystem eingesetzt wird. Beispielsweise kann über das Steuergerät dann ein Rollladen geöffnet oder geschlossen bzw. eine Innenbeleuchtung leistungsgesteuert werden. Die Steuerung der haustechnischen Einrichtung mit dem Steuergerät kann beispielsweise über WLAN oder Bluetooth erfolgen, so dass eine Bedienperson während einer Bedienung des Anzeigegeräts mit dem Steuergerät bequem Lichtverhältnisse im Untersuchungsraum zur Durchführung bestimmter Sehtests steuern kann. Unter anderem kann auch vorgesehen sein, dass bei einer Anwahl eines bestimmten Sehtests durch eine Bedienperson am Steuergerät die haustechnischen Einrichtungen von dem Steuergerät automatisch zur Anpassung der Beleuchtungsbedingungen an den Sehtest angesteuert werden.

Prinzipiell kann der Bildschirm auch beispielsweise mit linearer oder zirkularer Polarisation, oder einer anderen Einrichtung, die zur Bildtrennung nutzbar ist, ausgebildet sein. Zu den Vorteilen des erfindungsgemäßen Verfahrens wird auf die Vorteilsbeschreibung des erfindungsgemäßen Sehprüfsystems verwiesen.

Mittels einer Kameravorrichtung des Anzeigegeräts kann ein Pupillendurchmesser gemessen werden, wobei ein Sehtest bei Dämmerungssehen, oder Nachtsehen eines Probanden durchgeführt werden kann. So wird es dann erst mittels einer Beleuchtung der Pupillen mit Infrarotlicht durch eine Beleuchtungsvorrichtung möglich, den Pupillendurchmesser zu messen und mittels eines Sehtests eine subjektive Refraktion bei mesopischen oder skotopischen Sehbedingungen zu bestimmen. Die subjektiven Refraktionswerte können dann mit bei im wesentlichen gleichen Pupillendurchmesser gemessenen objektiven Refraktionswerten verglichen werden. Die objektiven Refraktionswerte können beispielsweise automatisch von einem Messgerät zur Bestimmung von objektiven Refraktionswerten an das Sehprüfsystem übermittelt werden.

Weiter kann eine Anpassung einer Bildschirmleuchtdichte des Bildschirms an eine Umgebungsleuchtdichte erfolgen, wobei die Anpassung der Bildschirmleuchtdichte in Abhängigkeit der Umgebungsleuchtdichte proportional erfolgen kann. Bei dem Verfahren wird dann folglich mittels einer Anpassungsvorrichtung des Bildschirms eine Anpassung der Bildschirmleuchtdichte an die Umgebungsleuchtdichte so vorgenommen, dass ein Verhältnis zwischen Bildschirmleuchtdichte und Umgebungsleuchtdichte stets linear ist.

In einer Ausführungsform des Verfahrens kann die Bildschirmleuchtdichte und die Farbwiedergabe einer Anzeigefläche des Bildschirms mittels eines optoelektronischen Sensors gemessen und mittels einer Anpassungsvorrichtung des Bildschirms geregelt werden. Sehtests, die eine definierte Darstellung einer Farbe oder eines Kontrasts von Sehprüfzeichen erfordern, können dann besonders genau durchgeführt werden. Die Regelung der Bildschirmleuchtdichte bzw. der Farbwiedergabe kann automatisch durch die Anpassungsvorrichtung erfolgen, so dass der Bildschirm sich selbst kalibriert. Der Bildschirm kann beispielsweise ein Bildschirm eines Tablet-Computers oder eines herkömmlichen Fernsehgerätes sein. Eine Darstellung von Sehprüfzeichen kann bei einer Hintergrundbeleuchtung des Bildschirms von 90 bis 300 cd/m² Bildschirmleuchtdichte erfolgen. Eine Darstellung eines Hintergrunds der Sehprüfzeichen in Graustufen ist zwar möglich, jedoch nicht erforderlich, da die Bildschirmleuchtdichte über die Steuerung der Hintergrundbeleuchtung leicht anpassbar ist.

Mittels der Kameravorrichtung des Anzeigegeräts kann ein Pupillenabstand, ein Pupillendurchmesser, ein Messabstand, eine Kopfneigung und/oder eine Blickrichtung von Augen des Probanden erfasst und gemessen werden. Wenn ein Sehabstand des Probanden zu dem Bildschirm durch eine ortsfeste Platzierung von Bildschirm und Proband prinzipiell bekannt ist, kann mittels Bildverarbeitung aus einem mit der Kameravorrichtung bzw. einer Kamera aufgenommenen Bild beider Augen des Probanden der Pupillenabstand berechnet werden. Ein Relativabstand der Pupillen kann beispielsweise zur Anpassung einer Brille oder zur Darbietung bestimmter Sehtests genutzt werden. Der Pupillendurchmesser kann in Abhängigkeit einer Umgebungsbeleuchtung ebenfalls derart gemessen werden. Sofern ein Pupillenabstand bekannt sein sollte, kann umgekehrt mittels Bildverarbeitung aus einem mit der Kameravorrichtung aufgenommenen Bild ein Messabstand bzw. Sehabstand des Probanden relativ zum Bildschirm errechnet werden. Auch können eine Kopfneigung des Probanden relativ zum Bildschirm sowie eine Blickrichtung bzw. Fixation von Sehprüfzeichen erfasst werden.

So ist es auch besonders vorteilhaft, wenn mittels eines Lagesensors des Anzeigegeräts eine Position, insbesondere eine Neigung des Bildschirms relativ zu den Augen des Probanden gemessen werden kann. Der Lagesensor kann ein gyroskopischer Sensor sein, über den eine räumliche Position bzw. Lage des Bildschirms bzw. der Anzeigefläche bestimmbar ist. Wenn beispielsweise eine Aufnahme der Augen des Probanden oder dessen Kopfs mit einer Kameravorrichtung des Anzeigegeräts erfolgt, kann unter Einbeziehung eines bekannten Pupillenabstands leicht eine Neigung des Bildschirms relativ zu den Augen errechnet werden. Über den Bildschirm kann dann auch angezeigt werden, dass der Bildschirm relativ zu den Augen geneigt ist und beispielsweise kein Sehtest durchgeführt werden kann. Auch können über den Bildschirm Informationen zur richtigen Ausrichtung des Bildschirms relativ zu den Augen des Probanden ausgegeben werden. Der Proband ist so gegebenenfalls selbst in der Lage, den Bildschirm relativ zu seinen Augen in die für einen Sehtest erforderliche Position zu bringen.

Mittels der Kameravorrichtung des Anzeigegeräts kann auch eine kontinuierliche Blickerfassung von Augen des Probanden erfolgen. Demnach kann ein Fixationspunkt der Augen auf einer Anzeigefläche des Bildschirms errechnet werden. Dies ist dann möglich, wenn eine Blickrichtung der Augen des Probanden erfasst wird. So kann dann im Rahmen von Sehtests untersucht werden, inwieweit der Proband monokular oder binokular dargebotene Sehprüfzeichen dynamisch verfolgt.

Wenn die kontinuierliche Blickerfassung für dargebotene Sehprüfzeichen erfolgt, kann aus einer Wechselbeziehung von Augenbewegung und Sehzeichenposition eine monokulare und/oder binokulare Sehleistung bestimmt werden. Dieses sogenannte Eye-Tracking kann beispielsweise auch beim Lesen von auf dem Bildschirm dargebotenen Texten eingesetzt werden.

Wenn ein Sehabstand bzw. ein Messabstand bekannt ist, können die Sehprüfzeichen in einer an den Messabstand angepassten Größe dargestellt werden. Die Darstellung der größenangepassten Sehprüfzeichen kann automatisch oder manuell über das Steuergerät bzw. durch eine Bedienperson erfolgen. So werden die Sehprüfzeichen immer in der erforderlichen Größe dargestellt und Fehler bei einer Durchführung von Sehtests werden vermieden.

Das Sehprüfsystem kann als ein Anzeigegerät ein ortsfestes Ferntest-Anzeigegerät und ein mobil handhabbares Nahtest-Anzeigegerät aufweisen, wobei ein mit dem Ferntest-Anzeigegerät gemessener Pupillenabstand und/oder ein Pupillendurchmesser bei einer Messung mit dem Nahtest-Anzeigegerät verwendet werden kann. Wenn ein Proband vor dem Ferntest-Anzeigegerät in einem definierten Sehabstand bzw. Messabstand platziert wird, ist der Messabstand dann bekannt. Mittels einer Kameravorrichtung des Anzeigegeräts kann dann ein Pupillenabstand des Probanden gemessen werden. Der Pupillenabstand kann bei bekanntem Messabstand mittels Bildverarbeitung aus einem Kamerabild bestimmt werden. Ebenso ist es möglich, einen beleuchtungsabhängigen Pupillendurchmesser derart zu bestimmen. Der Pupillenabstand und/oder der Pupillendurchmesser kann bei einer Messung mit dem Nahtest-Anzeigegerät derart verwendet werden, dass ein Messabstand bzw. ein Sehabstand der Augen eines Probanden zu dem Bildschirm des Nahtest-Anzeigegeräts errechnet wird. Wenn das Nahtest-Anzeigegerät ebenfalls über eine Kameravorrichtung verfügt, dann kann mittels Bildverarbeitung aus einem Bild der Kameravorrichtung ein Pupillenabstand bzw. ein Pupillendurchmesser des Probanden erfasst und in Relation zu dem mit dem Ferntest-Anzeigegerät gemessenen Pupillenabstand und Pupillendurchmesser gesetzt werden, so dass in Relation zur Bildaufnahme des Nahtest-Anzeigegeräts bzw. dessen Kameravorrichtung der Messabstand berechnet werden kann. Derartige Berechnungen können beispielsweise mittels Triangulation erfolgen und von dem Steuergerät ausgeführt werden. Eine Darbietung von Sehprüfzeichen kann dann beispielsweise immer in Abhängigkeit eines tatsächlichen Messabstands des Nahtest-Anzeigegeräts auf dessen Bildschirm erfolgen.

Ein Messabstand von Augen eines Probanden relativ zu dem Bildschirm des Nahtest-Anzeigegeräts wird noch genauer bestimmbar, wenn der mit dem Ferntest-Anzeigegerät bei bekanntem Messabstand gemessene Pupillenabstand zur Entfernungsmessung mit einer Kameravorrichtung des Nahtest-Anzeigegeräts verwendet wird, wobei eine Konvergenz der Augen berücksichtigt werden kann. Da mit dem Nahtest-Anzeigegerät gegebenenfalls bei Messabständen von 10 cm bis 3 m keine Sehprüfzeichen mehr im Unendlichen betrachtet werden können, fokussieren die Augen des Probanden dann ein Sehprüfzeichen, welches auf dem Bildschirm des Nahtest-Anzeigegeräts dargeboten wird. Blickachsen der Augen verlaufen dabei im Wesentlichen konvergent. Dadurch ergibt sich ein gegenüber einem, bei einem Ferntest bzw. Blick in das Unendliche gemessenen Pupillenabstand ein verringerter Pupillenabstand, der bei einer Berechnung eines Messabstands und/oder der Durchführung von Sehtests berücksichtigt werden kann.

Mit der Visualisierung der Sehprüfzeichen kann ein Sehtest durchgeführt werden, wobei mit dem Sehtest ein Augenversatz, monokulares Sehen, binokulares Sehen, Tagsehen, Dämmerungssehen oder Nachtsehen eines Probanden bestimmt werden kann. Der Augenversatz kann beispielsweise mit einem Sehtest nach Maddox oder Thorington ermittelt werden. Bei dem monokularen bzw. binokularen Sehen kann eine Refraktion der Augen, auch in Verbindung mit einer Messbrille oder einem Phoropter ermittelt werden. Neben der Durchführung von Sehtests bei photopischem Licht- bzw. Beleuchtungsbedingungen können auch mesopische oder skotopische Licht- bzw. Beleuchtungsbedingungen eingestellt werden, um das Dämmerungssehen und Nachtsehen eines Probanden zu prüfen bzw. zu ermitteln. Dabei können dann insbesondere die Bildschirmleuchtdichte und die Umgebungsleuchtdichte so weit abgesenkt werden, dass mesopische oder skotopische Sehbedingungen geschaffen werden. Beispielsweise kann dann auch eine sogenannte Nachtbrille dem Probanden bei erweiterter Pupille angepasst werden.

Die subjektive Überprüfung der objektiv gemessenen Refraktionswerte kann unter Berücksichtigung eines Pupillendurchmessers erfolgen. Da gegebenenfalls die objektiv gemessenen Refraktionswerte bei einheitlichen Lichtverhältnissen mit beispielsweise einem kleinen Pupillendurchmesser mittels eines Aberrometers ermittelt wurden, kann bei der subjektiven Überprüfung die Bildschirmleuchtdichte und die Umgebungsleuchtdichte so weit vermindert werden, dass der Pupillendurchmesser vergleichsweise größer wird. Dann ergeben sich gegebenenfalls subjektiv gemessene Refraktionswerte, die von den objektiv gemessenen Refraktionswerten abweichen.

Mit der Visualisierung der Sehprüfzeichen kann eine Phorie eines Probanden bestimmt werden, wobei die Phorie allein durch eine Verschiebung von zumindest einem allein vom rechten Auge wahrnehmbaren Sehprüfzeichen und zumindest einem allein vom linken Auge wahrnehmbaren Sehprüfzeichen relativ zueinander bestimmt werden kann. Die Bestimmung einer dissoziierten Phorie kann mit einem Sehtest nach Maddox oder Thorington erfolgen. Dabei können außerhalb des Bildschirms, beispielsweise in einem Rahmen des Anzeigegeräts angeordnete Leuchtmittel wie Leuchtdioden in Verbindung mit einer vom Bildschirm dargestellten Skala verwendet werden. Auch kann dadurch, dass Sehprüfzeichen vom rechten und linken Auge gegebenenfalls getrennt wahrnehmbar und bewegbar sind, unter Zuhilfenahme einer Messbrille oder eines Phoropters mit einer Polarisationsfolie, durch eine Relativverschiebung eine Phorie bestimmt werden, ohne dass Prismen verwendet werden müssten.

Weiter kann die Umweltsituation eine Verkehrssituation bei Sonnenschein, Nebel, Regen, Dämmerung oder Nacht mit oder ohne Darstellung künstlicher Beleuchtung sein. Beispielsweise kann ein Fahrzeug auf einer Straße dargestellt werden, wobei dann Sehprüfzeichen in ein Kfz-Nummernschild eingebettet sein können. Die Bildwiedergabe kann dann so weit vergrößert oder verkleinert werden, dass sich beispielsweise ein größerer subjektiver Sehabstand zu der dargestellten Umweltsituation bzw. dem Fahrzeug ergibt. So kann beispielsweise leicht überprüft werden, ob ein Proband noch in der Lage ist, innerhalb eines frei wählbaren Abstands zu dem Fahrzeug das Kfz-Nummernschild des Fahrzeugs zu erkennen. Dieser Sehtest kann wie zuvor beschrieben mit anderen Beleuchtungsbedingungen variiert werden.

Auch können Lichter eines Fahrzeugs dargestellt werden, wobei eine Farbfehlsichtigkeit durch Variation eines Farbtons der Lichter bestimmt werden kann. Die Lichter des Fahrzeugs können beispielsweise Rückleuchten oder Bremsleuchten sein, so dass ein Farbton unabhängig voneinander variiert wird. Beispielsweise kann der Farbton ein Rotton sein, so dass eine Farbwahrnehmung dieses Farbtons durch zwei unterschiedlich variierte Lichter überbrückt werden kann.

Die Umweltsituation kann auch dreidimensional wahrnehmbar dargestellt werden. Beispielsweise kann der Bildschirm von einem herkömmlichen Fernsehgerät ausgebildet werden, welches zur dreidimensionalen Wiedergabe in Verbindung mit beispielsweise einer Polarisationsbrille bzw. Messbrille mit Polarisationsfilter geeignet ist. Gleichwohl kann vorgesehen sein, die Umweltsituation zweidimensional darzustellen. Besonders vorteilhaft ist es, wenn ein Bildschirm verwendet wird, der die Umweltsituation und/oder die Sehprüfzeichen im 4K-Format darstellen kann.

Weitere Ausführungsformen des Verfahrens ergeben sich aus den auf den Vorrichtungsanspruch 1 rückbezogenen Unteransprüchen.

Nachfolgend wird eine bevorzugte Ausführungsform der Erfindung unter Bezugnahme auf die beigefügten Zeichnungen näher erläutert.

Es zeigen:
- **Fig. 1**: eine schematische Darstellung einer Ausführungsform eines Sehprüfsystems;
- **Fig. 2**: eine schematische Darstellung einer Anordnung eines Sehprüfsystems.

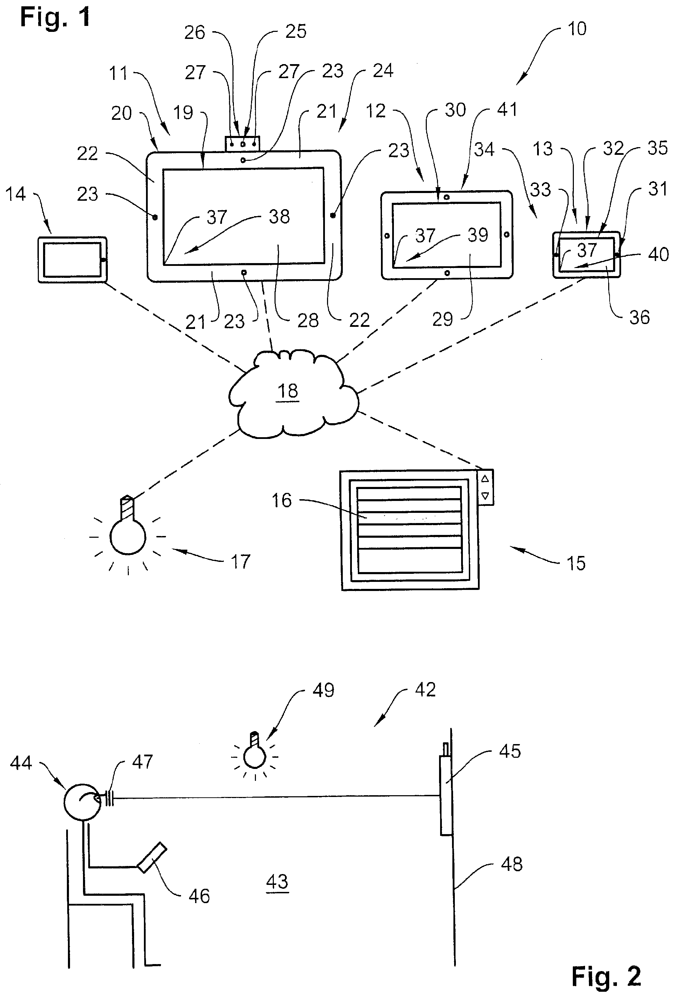

Die **Fig. 1** zeigt ein Sehprüfsystem 10, umfassend ein Ferntest-Anzeigegerät 11, ein weiteres Ferntest-Anzeigegerät 12 und ein Nahtest-Anzeigegerät 13 sowie ein Steuergerät 14 zur Steuerung der Anzeigegeräte 11, 12 bzw. 13. Weiter umfasst das Sehprüfsystem 10 eine Rollladensteuerung 15 mit einem Rollladen 16 und eine steuerbare Lichtquelle 17 eines hier nicht dargestellten Raums. Die Rollladensteuerung 15 und die Lichtquelle 17 sind ebenfalls mit dem Steuergerät 14 steuerbar. Die Anzeigegeräte 11, 12 und 13 sowie die Rollladensteuerung 15 und die Lichtquelle 17 sind über ein WLAN-Netzwerk 18 zum Austausch von Daten mit dem Steuergerät 14 verbunden. Das Ferntest-Anzeigegerät 11 ist aus einem Bildschirm 19 mit einem Rahmen 20 ausgebildet. Der Bildschirm 19 ist hintergrundbeleuchtet und kann linear oder zirkular polarisiert sein. In Längsseiten 21 und 22 des Rahmens 20 sind jeweils Leuchtdioden 23 integriert, die zusammen eine Blendvorrichtung 24 ausbilden. Mittels der Leuchtdioden 23 können Augen eines Probanden so beleuchtet werden, dass eine Blendwirkung erzielt wird. Das Ferntest-Anzeigegerät 11 weist weiter eine Kameravorrichtung 25 und eine Beleuchtungsvorrichtung 26 mit Infrarotleuchtdioden 27 auf. Die Kameravorrichtung 25 kann zusammen mit der Beleuchtungsvorrichtung 26 in den Rahmen 20 motorisch abgesenkt werden. Eine Anzeigefläche 28 des Bildschirms 11 ist vergleichsweise größer ausgebildet als eine Anzeigefläche 29 eines Bildschirms 30 des Ferntest-Anzeigegeräts 12. Das Ferntest-Anzeigegerät 11 kann daher für vergleichsweise größere Messabstände bzw. Sehabstände von Sehtests als das Ferntest-Anzeigegerät 12 genutzt werden.

Das Nahtest-Anzeigegerät 13 verfügt ebenfalls über eine Kameravorrichtung 31 in einem Rahmen 32 sowie über eine Leuchtdiode 33, die eine Blendvorrichtung 34 ausbildet. Eine Anzeigefläche 36 eines Bildschirms 35 des Nahtest-Anzeigegeräts 13 ist vergleichsweise klein gegenüber der Anzeigefläche 29 des Ferntest-Anzeigegeräts 12. Hier nicht dargestellte Sehzeichen bzw. Sehtests können über das Steuergerät 14 auf den jeweiligen Anzeigeflächen 28, 29 und 36 wahlweise dargestellt werden. Weiter weisen die Anzeigegeräte 11, 12 und 13 jeweils optoelektronische Sensoren 37 auf, die in einer Ecke 38, 39 und 40 der Rahmen 20, 32 und 41 angeordnet sind. Die optoelektronischen Sensoren 37 sind Teil einer hier nicht näher dargestellten Messeinrichtung, mit der jeweils eine Bildschirmleuchtdichte der Bildschirme 19, 30 bzw. 35 sowie eine Umgebungsleuchtdichte eines Raums gemessen werden. Eine Messung der Umgebungsleuchtdichte erfolgt mittels eines hier nicht dargestellten optoelektronischen Sensors, der jeweils in den Rahmen 20, 32 und 41 integriert ist. Die Bildschirmleuchtdichte wird dann mittels des Steuergeräts 14 oder durch das Anzeigegerät 11, 12 bzw. 13 an die Umgebungsleuchtdichte angepasst, wobei die Anpassung proportional erfolgt. Die Umgebungsleuchtdichte ist ihrerseits durch Manipulation des Rollladen 16 und der Lichtquelle 17 über das Steuergerät 14 manuell oder automatisch einstellbar.

Die **Fig. 2** zeigt ein Sehprüfsystem 42 in einem Raum 43 zusammen mit einem Probanden 44. Das Sehprüfsystem 42 umfasst ein Ferntest-Anzeigegerät 45 und ein Nahtest-Anzeigegerät 46 sowie eine Messbrille 47, die der Proband 44 trägt. Das Ferntest-Anzeigegerät 45 ist ortsfest an einer Wand 48 montiert und das Nahtest-Anzeigegerät 46 wird von dem Probanden 44 manuell gehalten. Weiter ist der Raum 43 mit einer Lichtquelle 49 ausgestattet, über die eine Umgebungsleuchtdichte steuerbar ist. Über ein hier nicht dargestelltes Steuergerät des Sehprüfsystems 42 kann von einer Bedienperson ein Sehtest mit dem Probanden 44 durchgeführt werden, indem auf dem Ferntest-Anzeigegerät 45, welches sich in einem vergleichsweise großem Messabstand relativ zu dem Probanden 44 befindet, Sehprüfzeichen dargeboten werden. Das Nahtest-Anzeigegerät 46 kann von dem Probanden 44 selbst gehandhabt werden, wobei hier ein Messabstand zwischen dem Probanden 44 und dem Nahtest-Anzeigegerät 46 vergleichsweise kleiner ist. Auch hier können nach Bedarf dem Probanden 44 über das Steuergerät Sehprüfzeichen dargeboten werden.

## Patentansprüche

1. Verfahren zum Überprüfen der Augen eines Probanden mit einem Sehprüfsystem (10, 42), wobei mit einem Anzeigegerät (11, 12, 13, 45, 46) des Sehprüfsystems zumindest einem Auge des Probanden (44) visualisierte Sehprüfzeichen angezeigt werden, wobei mit der Visualisierung der Sehprüfzeichen ein Sehtest durchgeführt wird, bei dem die Sehprüfzeichen in eine Bildwiedergabe einer realen Umweltsituation eingebettet sind, wobei das Anzeigegerät mit einem Steuergerät (14) des Sehprüfsystems gesteuert wird, wobei das Anzeigegerät einen hintergrundbeleuchteten Bildschirm (19, 30, 35) umfasst, wobei das Anzeigegerät eine Beleuchtungsvorrichtung (26) mit einer Infrarotlichtquelle (27) aufweist, wobei das Anzeigegerät eine der Infrarotlichtquelle entsprechend angepasste Kameravorrichtung (25, 31) aufweist, wobei mittels der Infrarotlichtquelle Augen des Probanden mit Infrarotlicht beleuchtet und mittels der Kameravorrichtung unabhängig von einer Umgebungsbeleuchtung aufgenommen werden, wobei objektiv gemessene Refraktionswerte des Probanden an das Steuergerät übermittelt oder in dieses eingegeben werden, wobei das Steuergerät die Größe der darzustellenden Sehprüfzeichen in Abhängigkeit der objektiven Refraktionswerte automatisch auswählt, wobei die Sehprüfzeichen in einer Größe visualisiert werden, die an die objektiv gemessenen Refraktionswerte des Probanden angepasst sind, wobei eine subjektive Überprüfung der objektiv gemessenen Refraktionswerte erfolgt.

2. Verfahren nach Anspruch 1,
**dadurch gekennzeichnet,**
**dass** mittels der Kameravorrichtung (25, 31) des Anzeigegeräts (11, 12, 13, 45, 46) ein Pupillendurchmesser gemessen wird, wobei ein Sehtest bei Dämmerungssehen, oder Nachtsehen eines Probanden (44) durchgeführt wird.

3. Verfahren nach Anspruch 1 oder 2,
**dadurch gekennzeichnet,**
**dass** eine Anpassung einer Bildschirmleuchtdichte des Bildschirms an eine Umgebungsleuchtdichte erfolgt, wobei die Anpassung der Bildschirmleuchtdichte in Abhängigkeit der Umgebungsleuchtdichte proportional erfolgt.

4. Verfahren nach einem der Ansprüche 1 bis 3,
**dadurch gekennzeichnet,**
**dass** mittels der Kameravorrichtung (25, 31) des Anzeigegeräts (11, 12, 13, 45, 46) ein Pupillenabstand, ein Pupillendurchmesser, ein Messabstand, eine Kopfneigung und/oder eine Blickrichtung von Augen des Probanden (44) erfasst und gemessen wird.

5. Verfahren nach einem der Ansprüche 1 bis 4,
**dadurch gekennzeichnet,**
**dass** mittels eines Lagesensors des Anzeigegeräts (11, 12, 13, 45, 46) eine Position, insbesondere eine Neigung des Bildschirms (19, 30, 35) relativ zu den Augen des Probanden (44) gemessen wird.

6. Verfahren nach einem der Ansprüche 1 bis 5,
**dadurch gekennzeichnet,**
**dass** mittels der Kameravorrichtung (25, 31) des Anzeigegeräts (11, 12, 13, 45, 46) eine kontinuierliche Blickerfassung von Augen des Probanden (44) erfolgt.

7. Verfahren nach Anspruch 6,
**dadurch gekennzeichnet,**
**dass** die kontinuierliche Blickerfassung für dargebotene Sehprüfzeichen erfolgt, wobei aus einer Wechselbeziehung von Augenbewegung und Sehzeichenposition eine monokulare und/oder binokulare Sehleistung bestimmt wird.

8. Verfahren nach einem der Ansprüche 1 bis 7,
**dadurch gekennzeichnet,**
**dass** die Sehprüfzeichen in einer an den Messabstand angepassten Größe dargestellt werden.

9. Verfahren nach einem der Ansprüche 1 bis 8,
**dadurch gekennzeichnet,**
**dass** das Sehprüfsystem (10, 42) als ein Anzeigegerät ein ortsfestes Ferntest-Anzeigegerät (11, 12, 45) und ein mobil handhabbares Nahtest-Anzeigegerät (13, 46) aufweist, wobei ein mit dem Ferntest-Anzeigegerät gemessener Pupillenabstand und/oder ein Pupillendurchmesser bei einer Messung mit dem Nahtest-Anzeigegerät verwendet wird.

10. Verfahren nach Anspruch 9,
**dadurch gekennzeichnet,**
**dass** der mit dem Ferntest-Anzeigegerät (11, 12, 45) bei bekannten Messabstand gemessene Pupillenabstand zur Entfernungsmessung mit der Kameravorrichtung (31) des Nahtest-Anzeigegeräts (13, 46) verwendet wird, wobei eine Konvergenz der Augen berücksichtigt wird.

11. Verfahren nach einem der Ansprüche 1 bis 10,
**dadurch gekennzeichnet,**
**dass** mit der Visualisierung der Sehprüfzeichen ein Sehtest durchgeführt wird, wobei mit dem Sehtest ein Augenversatz, monokulares Sehen, binokulares Sehen, Tagsehen, Dämmerungssehen, oder Nachtsehen eines Probanden (44) bestimmt wird.

12. Verfahren nach einem der Ansprüche 1 bis 11,
**dadurch gekennzeichnet,**
**dass** die subjektive Überprüfung der objektiv gemessenen Refraktionswerte unter Berücksichtigung eines Pupillendurchmessers erfolgt.

13. Verfahren nach einem der Ansprüche 1 bis 12,
**dadurch gekennzeichnet,**
**dass** mit der Visualisierung der Sehprüfzeichen eine Phorie eines Probanden (44) bestimmt wird, wobei die Phorie alleine durch eine Verschiebung von zumindest ein alleine vom rechten Auge wahrnehmbaren Sehprüfzeichen und zumindest ein alleine vom linken Auge wahrnehmbaren Sehprüfzeichen relativ zueinander bestimmt wird.

14. Verfahren nach einem der Ansprüche 1 bis 13,
**dadurch gekennzeichnet,**
**dass** die Umweltsituation eine Verkehrssituation bei Sonnenschein, Nebel, Regen, Dämmerung oder Nacht mit oder ohne Darstellung künstlicher Beleuchtung ist.

15. Verfahren nach einem der Ansprüche 1 bis 14,
**dadurch gekennzeichnet,**
**dass** Lichter eines Fahrzeugs dargestellt werden, wobei eine Farbfehlsichtigkeit durch Variation eines Farbtons der Lichter bestimmt wird.

## Claims

1. A method for testing the eyes of a subject by means of a vision testing system (10, 42), optotypes visualized to at least one eye of the subject (44) being displayed by means of a display device (11, 12, 13, 45, 46) of the vision testing system, an eyesight test being performed by visualizing the optotype, in which the optotypes are embedded in an image replication of a real environmental situation, the display device being controlled by means of a control device (14) of the vision testing system, the display device comprising a backlit screen (19, 30, 35), the display device having an illuminating device (26) comprising an infrared light source (27), the display device having a camera device (25, 31) correspondingly adapted to the infrared light source, eyes of the subject being illuminated with infrared light by means of the infrared light source and captured by means of the camera device independently of an ambient illumination, objectively measured refraction values of the subject being transmitted to the control device or entered into the same, the control device automatically selecting the size of the optotypes to be displayed as a function of the objective refraction values, the optotypes being visualized in a size that is adapted to the objectively measured refraction values of the subject, the objectively measured refraction values being subjectively reviewed.

2. The method according to claim 1,
**characterized in that**
a pupillary distance is measured by means of the camera device (25, 31) of the display device (11, 12, 13, 45, 46), an eyesight test being performed under mesopic vision or scotopic vision of a subject (44).

3. The method according to claim 1 or 2,
**characterized in that**
a screen luminance of the screen is adjusted to an ambient luminance, the screen luminance being adjusted proportionally as a function of the ambient luminance.

4. The method according to any one of claims 1 to 3,
**characterized in that**
a pupillary distance, a pupil diameter, a measuring distance, a head tilt and/or a line of sight of the eyes of the subject (44) is registered and measured by means of the camera device (25, 31) of the display device (11, 12, 13, 45, 46).

5. The method according to any one of claims 1 to 4,
**characterized in that**
a position, in particular a tilt of the screen (19, 30, 35) relative to the eyes of the subject (44) is measured by means of a position sensor of the display device (11, 12, 13, 45, 46).

6. The method according to any one of claims 1 to 5,
**characterized in that**
the eyes of the subject (44) are continuously tracked by means of the camera device (25, 31) of the display device (11, 12, 13, 45, 46).

7. The method according to claim 6,
**characterized in that**
the continuous eye tracking takes place for presented optotypes, a monocular and/or binocular visual performance being determined from an interrelation between eye movement and optotype position.

8. The method according to any one of claims 1 to 7,
**characterized in that**
the optotypes are presented in a size adjusted to the measuring distance.

9. The method according to any one of claims 1 to 8,
**characterized in that**
for a display device, the vision testing system (10, 42) has a stationary distance-test display device (11, 12, 45) and a mobile near-test display device (13, 46), a pupillary distance and/or a pupil diameter measured with the distance-test display device being used when measuring with the near-test display device.

10. The method according to claim 9,
**characterized in that**
the pupillary distance measured with the distance-test display device (11, 12, 45) at a known measuring distance is used for distance measuring with the camera device (31) of the near-test display device (13, 46), a convergence of the eyes being taken into account.

11. The method according to any one of claims 1 to 10,
**characterized in that**
an eyesight test is performed by visualizing the optotypes, the eyesight test determining eye offset, monocular vision, binocular vision, photopic vision, mesopic vision or scotopic vision of a subject (44).

12. The method according to any one of claims 1 to 11,
**characterized in that**
the objectively measured refraction values are subjectively reviewed, the review taking a pupil diameter into account.

13. The method according to any one of claims 1 to 12,
**characterized in that**
by visualizing the optotypes, a phoria of a subject (44) is determined, the phoria being determined solely by shifting at least one optotype visible the right eye alone and by shifting at least one optotype visible to the left eye alone relative to each other.

14. The method according to any one of claims 1 to 13,
**characterized in that**
the environmental situation is a traffic situation in sunshine, fog, rain, twilight or at night with and without display of artificial lighting.

15. The method according to any one of claims 1 to 14,
**characterized in that**
the lights of a vehicle are shown, a color vision deficiency being determined by varying a shade of color of the lights.

## Revendications

1. Procédé d'examen des yeux d'un sujet au moyen d'un système de test de vue (10, 42), des optotypes visualisés à au moins un œil du sujet (44) étant affichés au moyen d'un dispositif d'affichage (11, 12, 13, 45, 46) du système de test de vue, un test de vue étant effectué par la visualisation des optotypes, dans lequel les optotypes sont intégrés dans une reproduction d'image d'une situation environnementale réelle, le dispositif d'affichage étant commandé au moyen d'un dispositif de commande (14) du système de test de vue, le dispositif d'affichage comprenant un écran rétroéclairé (19, 30, 35), le dispositif d'affichage ayant un dispositif d'éclairage (26) comprenant une source de lumière infrarouge (27), le dispositif d'affichage ayant un dispositif de caméra (25, 31) adapté de manière correspondante à la source de lumière infrarouge, des yeux du sujet étant éclairés par la lumière infrarouge au moyen de la source de lumière infrarouge et capturés au moyen du dispositif de caméra indépendamment d'un éclairage ambiant, des valeurs de réfraction mesurées objectivement du sujet étant transmises au dispositif de commande ou entrées dans celui-ci, le dispositif de commande sélectionnant automatiquement la taille des optotypes à afficher en fonction des valeurs de réfraction objectives, les optotypes étant visualisés dans une taille adaptée aux valeurs de réfraction mesurées objectivement du sujet, les valeurs de réfraction mesurées objectivement étant examinées subjectivement.

2. Procédé selon la revendication 1,
**caractérisé en ce**
**qu'**une distance pupillaire est mesurée au moyen du dispositif de caméra (25, 31) du dispositif d'affichage (11, 12, 13, 45, 46), un test de vue étant effectué sous vision mésopique ou vision scotopique d'un sujet (44).

3. Procédé selon la revendication 1 ou 2,
**caractérisé en ce**
**qu'**une luminance de l'écran est ajustée à une luminance ambiante, la luminance de l'écran étant ajustée proportionnellement en fonction de la luminance ambiante.

4. Procédé selon l'une quelconque des revendications 1 à 3,
**caractérisé en ce**
**qu'**une distance pupillaire, un diamètre pupillaire, une distance de mesure, une inclinaison de la tête et/ou une ligne de visée des yeux du sujet (44) sont enregistrés et mesurés au moyen du dispositif de caméra (25, 31) du dispositif d'affichage (11, 12, 13, 45, 46).

5. Procédé selon l'une quelconque des revendications 1 à 4,
**caractérisé en ce**
**qu'**une position, notamment une inclinaison de l'écran (19, 30, 35) par rapport aux yeux du sujet (44) est mesurée au moyen d'un capteur de position du dispositif d'affichage (11, 12, 13, 45, 46).

6. Procédé selon l'une quelconque des revendications 1 à 5,
**caractérisé en ce**
**qu'**un regard des yeux du sujet (44) est suivi en continu au moyen du dispositif de caméra (25, 31) du dispositif d'affichage (11, 12, 13, 45, 46).

7. Procédé selon la revendication 6,
**caractérisé en ce que**
le suivi de regard continu est effectué pour des optotypes présentés, une performance visuelle monoculaire et/ou binoculaire étant déterminée à partir d'une interrelation entre le mouvement des yeux et la position des optotypes.

8. Procédé selon l'une quelconque des revendications 1 à 7,
**caractérisé en ce que**
les optotypes sont présentés dans une taille ajustée à la distance de mesure.

9. Procédé selon l'une quelconque des revendications 1 à 8,
**caractérisé en ce que**
le système de test de vue (10, 42) a un dispositif d'affichage de test de distance (11, 12, 45) fixe et un dispositif d'affichage de test de près (13, 46) mobile comme dispositif d'affichage, une distance pupillaire et/ou un diamètre pupillaire mesurés avec le dispositif d'affichage de test de distance étant utilisés lors d'une mesure avec le dispositif d'affichage de test de près.

10. Procédé selon la revendication 9,
**caractérisé en ce que**
la distance pupillaire mesurée avec le dispositif d'affichage de test de distance (11, 12, 45) à une distance de mesure connue est utilisée pour la mesure de distance avec le dispositif de caméra (31) du dispositif d'affichage de test de près (13, 46), une convergence des yeux étant prise en compte.

11. Procédé selon l'une quelconque des revendications 1 à 10,
**caractérisé en ce**
**qu'**un test de vue est effectué par la visualisation des optotypes, le test de vue déterminant un décalage oculaire, la vision monoculaire, la vision binoculaire, la vision photopique, la vision mésopique ou la vision scotopique d'un sujet (44).

12. Procédé selon l'une quelconque des revendications 1 à 11,
**caractérisé en ce que**
les valeurs de réfraction mesurées objectivement sont examinées subjectivement, l'examen tenant compte du diamètre de la pupille.

13. Procédé selon l'une quelconque des revendications 1 à 12,
**caractérisé en ce que**
par la visualisation des optotypes, on détermine une phorie d'un sujet (44), la phorie étant déterminée uniquement en décalant au moins un optotype visible uniquement par l'œil droit et en décalant au moins un optotype visible uniquement par l'œil gauche l'un par rapport à l'autre.

14. Procédé selon l'une quelconque des revendications 1 à 13,
**caractérisé en ce que**
la situation environnementale est une situation de circulation sous le soleil, dans le brouillard, sous la pluie, au crépuscule ou de nuit, avec ou sans représentation d'un éclairage artificiel.

15. Procédé selon l'une quelconque des revendications 1 à 14,
**caractérisé en ce que**
des feux d'un véhicule sont représentés, une anomalie de la vision des couleurs étant déterminée en faisant varier une teinte des feux.
